# EUROPÄISCHE PATENTANMELDUNG

(11) **EP 0 582 032 A1**
(43) Veröffentlichungstag der Anmeldung: **09.02.1994**
(21) Anmeldenummer: 93102902.9
(22) Anmeldetag: 25.02.1993
(51) Int. Cl.: A61M 3/02

(54) **Elektrische Irregationsvorrichtung**

(30) Priorität: 06.08.1992 DE 9210501 U
(71) Anmelder: B. Braun Melsungen AG, D-34209 Melsungen (DE)
(72) Erfinder: Schaefers, Heinz, W-4282 Velen-Ramsdorf (DE); Ziebarth, Harald, W-3509 Malsfeld (DE)
(74) Vertreter: Selting, Günther, Dipl.-Ing.

(57) **Zusammenfassung**

Die Irrigationsvorrichtung weist einen Flüssigkeitsbehälter (11) auf, in den vorgewärmte Spülflüssigkeit eingefüllt werden kann. An einer Wand (41) des Flüssigkeitsbehälters (11) ist ein Schaltungsgehäuse (12) befestigt, das an einem abnehmbaren Deckel (25) eine Schaltungsplatine (35) mit den elektrischen Komponenten enthält. In einem unter dem Flüssigkeitsbehälter (11) angeordneten Aufnahmeraum (44) befinden sich eine wiederaufladbare Batterie (46) und eine Pumpe (47). Im Behälterboden (43) ist ein Auslaß (49) vorgesehen, durch den von der Pumpe (47) Flüssigkeit zu einem Schlauchkonnektor (13) abgeführt wird.

## Beschreibung

Die Erfindung betrifft eine elektrische Irrigationsvorrichtung zur Einleitung einer Spülflüssigkeit in einen künstlichen Dickdarmausgang eines Patienten zum Zwecke einer Darmentleerung.

Bei schwerwiegender Darmerkrankung, die eine Schließung des Afterschließorgans erforderlich macht, werden Patienten mit einem künstlichen Darmausgang (Colostoma oder auch Stoma) durch die Bauchdecke hindurch versehen. Normalerweise wird an den künstlichen Darmausgang ein Beutelsystem angeschlossen, in das sich der Darm entleeren kann. Das ständige Tragen des Beutelsystems am Körper ist für Patienten oft beschwerlich und unangenehm. Hinzu kommen Probleme mit der Beutelentleerung. Um eine kontrollierte Darmentleerung mit nachfolgender stuhlgangfreier Zeit von 24 bis 48 Stunden zu erreichen, kann eine Irrigationsvorrichtung benutzt werden. Bei der Irrigation wird eine bestimmte Menge körperwarmen Wassers durch das Stoma hindurch in den Darm eingeführt. Dadurch wird die Darmwand gedehnt, so daß der Darm zur Tätigkeit und damit zur Entleerung angeregt wird. Durch diese Spülung erfolgt eine Stuhlentleerung mit anschließender sicherer Stuhlpause.

Bekannt ist eine Irrigationsvorrichtung (Prospekt "Irrigation" der Firma Dipl.-Ing. H. Schaefers, 4282 Velen-Ramsdorf), die einen Flüssigkeitsbehälter aufweist, an den seitlich ein Schaltungsgehäuse angesetzt ist. Das Schaltungsgehäuse enthält eine Kreiselpumpe, deren Einlaß an ein Schlauchstück angeschlossen ist, welches durch die Wand des Flüssigkeitsbehälter hindurchführt und über dem Behälterboden endet. Die Kreiselpumpe saugt aus dem Flüssigkeitsbehälter Spülflüssigkeit an und fördert sie zu einem Schlauchkonnektor, der seitlich von einer Wand des Schaltungsgehäuses absteht. Das Schaltungsgehäuse enthält ferner eine wiederaufladbare Batterie, sowie einen Ein/Ausschalter für die Kreiselpumpe, einen Drehknopf für die Pumpendrehzahl, eine Kontrolleuchte sowie eine Anschlußbuchse für ein externes Netzgerät zum Aufladen der Batterie. An den Schlauchkonnektor wird ein mit einem Beutel verbindbarer Spülschlauch angeschlossen. Der Beutel weist an einer Wand eine gelochte Platte auf, die derart am Patientenkörper befestigt werden kann, daß sie das Stoma dichtend umgibt und sie ist an der gegenüberliegenden Wand mit einem in das Stoma einsteckbaren Konus versehen, der mit dem Spülschlauch verbunden wird, um Spülflüssigkeit in den Darm einzuleiten.

Der Erfindung liegt die Aufgabe zugrunde, eine verbesserte elektrische Irrigationsvorrichtung zu schaffen, bei der konstruktive Aufbau vereinfacht ist und die im aufgestellten Zustand eine völlige Entleerung des Flüssigkeitsbehälters ermöglicht.

Aus EP 0 482 303 A1 ist eine Irrigationsvorrichtung bekannt, bei der eine Tauchpumpe im Innern eines Flüssigkeitsbehälters angeordnet ist. Seitlich neben dem Flüssigkeitsbehälter befindet sich ein Aufnahmeraum, der einen Transformator enthält und in den der Druckauslaß der Tauchpumpe hineinführt. Der Aufnahmeraum dient ferner zur Unterbringung des Spülschlauchs, wenn dieser nicht benutzt wird. Ein Schalter zum Einschalten des Geräts befindet sich an der Oberseite des Aufnahmeraums.

Die Lösung dieser Aufgabe erfolgt erfindungsgemäß mit den Merkmalen des Anspruchs 1.

Bei der erfindungsgemäßen Irrigationsvorrichtung ist unter dem Flüssigkeitsbehälter ein Aufnahmeraum vorgesehen, der die Kreiselpumpe und die Batterie enthält. Diese Komponenten sind somit nicht in dem seitlich am Flüssigkeitsbehälter vorgesehenen Schaltungsgehäuse enthalten, sondern unterhalb des hochgelegten Flüssigkeitsbehälters. Damit kann das Schaltungsgehäuse kleiner und übersichtlicher gestaltet werden. Insbesondere nimmt die relativ sperrige Batterie keinen Platz im Schaltungsgehäuse ein, so daß die im Schaltungsgehäuse enthaltenen Komponenten mehr Platz haben. Bei Wartung und Reparatur sind die im Schaltungsgehäuse enthaltenen Komponenten leichter zugänglich. Im Schaltungsgehäuse ist ferner nur eine einzige Verbindung eines wasserführenden Schlauchs erforderlich, nämlich die Verbindung des von der Pumpe kommenden Schlauchs mit dem Schlauchkonnektor. Die anderen Schlauchanschlüsse befinden sich in dem Aufnahmeraum, der von dem Raum des Schaltungsgehäuses getrennt ist, so daß eventuelle Leckagen die elektrische Schaltung nicht beeinträchtigen können. Durch das Höherlegen des Bodens des Flüssigkeitsbehälters kann der Flüssigkeitsbehälter vollständig in den Auslaß hinein entleert werden, so daß keine Restflüssigkeit in ihm stehen bleibt. Gemäß einer bevorzugten Ausführungsform der Erfindung weist das Schaltungsgehäuse einen abnehmbaren Deckel auf, an dem eine Schaltungsplatine befestigt ist. Diese Schaltungsplatine trägt die für die Pumpensteuerung und die Anzeigen erforderlichen elektrischen und elektronischen Komponenten und sie bildet gewissermaßen die Schaltungszentrale der Vorrichtung. Nach dem Entfernen des Deckels, hinter dem die Schaltungsplatine mit Abstand befestigt sind, ist die gesamte elektrische Schaltung zugänglich, so daß Messungen an verschiedenen Meßpunkten leicht durchgeführt und erforderlichenfalls elektrische Komponenten leicht ausgetauscht werden können. Die im Aufnahmeraum enthaltenen Komponenten sind durch elektrische Kabel mit der Schaltungsplatine verbunden, welche durch eine Öffnung in der Zwischenwand zwischen Schaltungsgehäuse und Aufnahmeraum hindurchgeführt sind. Durch diese Öffnung, die zweckmäßigerweise mit Abstand über dem gemeinsamen Boden von Schaltungsgehäuse und Aufnahmeraum angeordnet ist, kann auch der von der Pumpe zum Konnektor führende Pumpenschlauch hindurchgeführt sein.

Vorteilhafterweise trägt die Schaltungsplatine auch eine Steckbuchse für den Anschluß eines externen Netzgerätes an die Batterie. Auf diese Weise kann das Schaltungsgehäuse, einschließlich seines Deckels, sehr einfach ausgebildet sein, weil am Schaltungsgehäuse selbst keine Stecker oder Buchsen erforderlich sind.

Im folgenden wird unter Bezugnahme auf die Zeichnungen ein Ausführungsbeispiel der Erfindung näher erläutert.

Es zeigen:
- Fig. 1: eine perspektivische Darstellung der gesamten Irrigationsvorrichtung,
- Fig. 2: einen Querschnitt durch das Irrigationsgerät entlang der Linie II-II von Fig. 3 und
- Fig. 3: eine Draufsicht des Irrigationsgerätes aus Richtung des Pfeiles III von Fig. 1.

Die Irrigationsvorrichtung weist ein Irrigationsgerät 10 auf, das aus einem oben offenen Flüssigkeitsbehälter 11 und einem von einer Wand des Flüssigkeitsbehälters 11 abstehenden Schaltungsgehäuse 12 besteht. An dem Schaltungsgehäuse 12 befindet sich ein Schlauchkonnektor 13, der als Lüer-Lock-Konnektor ausgebildet ist und an dem mit einem entsprechenden Gegenkonnektor 14 ein Spülschlauch 15 angeschlossen werden kann, der zu einem Beutel 16 führt.

Der Beutel 16 ist ein langgestreckter flexibler Folienbeutel, der an seinem oberen Ende mit einer Schweißnaht 17 verschlossen ist und dessen unteres Ende offen ist und mit einer Klemmvorrichtung 18 wahlweise verschlossen werden kann. Im oberen Bereich des Schlauchs 16 befindet sich an einer Wand des Schlauchs eine Fixierplatte 19 mit einem Loch 20, die auf die Haut des Patienten derart aufgeklebt werden kann, daß das Loch 20 das Stoma umgibt. An der gegenüberliegenden Wand des Beutels ist ein Konus 21 befestigt, dessen Spitze zum Beutelinnern weist und durch das Loch 20 hindurch in das Stoma eingeführt werden kann. Der Konus 20 ist mit dem Spülschlauch 15 verbunden.

Das Schaltungsgehäuse 12 ist pultartig ausgebildet, d.h. mit einer nach außen schräg abfallenden Dachwand 22 versehen, von der der Schlauchkonnektor 13 rechtwinklig absteht. An der Dachwand 22 befindet sich ferner ein Drehknopf 23 zur Betätigung eines Ein/Aus-Schalters und gleichzeitig zur Steuerung der Pumpengeschwindigkeit. Zur Kennzeichnung der Drehknopfstellung ist der Drehknopf mit einer Markierung 23a versehen, die beim Drehen entlang farbig markierter Felder 24 bewegt wird, so daß anhand des Feldes, in dem sich die Markierung 23 befindet, die Schalter- bzw. Steuerstellung des Drehknopfes leicht erkannt werden kann. Diejenigen Teile, die bei Benutzung des Gerätes einer Handhabung zugänglich sein müssen, nämlich der Schlauchkonnektor 13 und der Drehknopf 23, befinden sich an der schrägen Dachwand 22, so daß sie bei aufgestelltem Irrigationsgerät leicht erkannt und bedient werden können. Die dem Flüssigkeitsbehälter 11 abgewandte Frontwand des Schaltungsgehäuses 12 ist als nach vorne abnehmbarer Deckel 25 ausgebildet, der insgesamt fünf Löcher aufweist, durch die farbige Kontrolleuchten 26 bis 30 hindurchragen. Die Kontrolleuchten 26, 27 und 28 zeigen die Temperatur der im Flüssigkeitsbehälter 11 enthaltenen Spülflüssigkeit an, wobei die Kontrolleuchte 34 leuchtet, wenn die Temperatur geringer ist als 34° C, die Kontrolleuchte 28 leuchtet, wenn die Temperatur größer ist als 38° C, und die Kontrolleuchte 27 leuchtet, wenn die Temperatur größer ist als 36° C. Da die Spülflüssigkeit Körpertemperatur haben sollte, sollte das Gerät nur dann in Betrieb genommen werden, wenn die (grüne) Kontrolleuchte 27 leuchtet und die Kontrolleuchten 26 und 28 erloschen sind.

Die Kontrolleuchte 29 leuchtet, wenn die im Innern des Gerätes enthaltene Batterie geladen wird, solange der Aufladezustand anhält, und die Kontrolleuchte 30 leuchtet, wenn das Gerät durch Betätigung des Drehknopfes 23 eingeschaltet ist.

Ein mit einem Netzstecker ausgestattetes Netzgerät 31 weist einen Stecker 32 auf, der an das Irrigationsgerät 10 angeschlossen werden kann. In dem Deckel 25 ist eine Öffnung 33 vorgesehen, durch die hindurch der Stecker 32 in eine Steckbuchse 34 im Innern des Schaltungsgehäuses eingeführt werden kann. Der Stecker 32 ist als Koaxialstecker ausgeführt und die Steckbuchse 34 ist ebenfalls eine Koaxial-Steckbuchse.

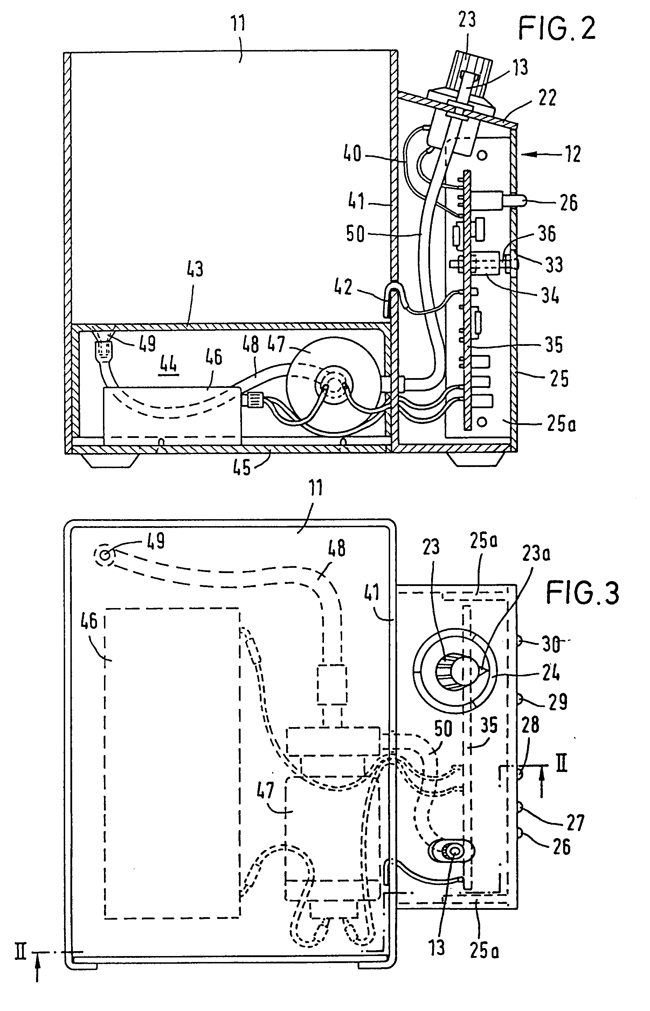

Gemäß Fig. 2 ist hinter dem Deckel 25 eine Schaltungsplatine 35 mit Schrauben 36 derart befestigt, daß die Schaltungsplatine mit Abstand von dem Deckel angeordnet und starr mit diesem verbunden ist. Der Deckel 25 weist Seitenwangen 25a auf, die zwischen die Seitenwände des Schaltungsgehäuses 12 passen und an diesen Seitenwänden mit Schrauben befestigt sind.

Die Schaltungsplatine 35 trägt die Anzeige- und Kontrolleuchten 26 bis 30 sowie die Steckbuchse 34. Sie trägt außerdem die erforderlichen elektrischen und elektronischen Komponenten. An die Leiterbahnen der Schaltungsplatine sind verschiedene Kabel angelötet, z.B. die von dem Gehäuse des Drehknopfs 23 kommenden Kabel 40.

In der Wand 41, die das Schaltungsgehäuse von dem Innern des Flüssigkeitsbehälters 11 trennt, ist in bodennähe des Flüssigkeitsbehälters ein Loch vorgesehen, durch das ein Temperaturfühler 42 abdichtend hindurchführt. Dieser Temperaturfühler 42, der zur Messung der Temperatur der Spülflüssigkeit dient, ist ebenfalls mit der Schaltungsplatine 35 verbunden.

Unter dem Boden 43 des Flüssigkeitsbehälters 11 befindet sich ein Aufnahmeraum 44, dessen Boden 45 den Geräteboden bildet. Der Aufnahmeraum 44 enthält eine auf dem Boden 45 befestigte wiederaufladbare Batterie 46 und eine als Kreiselpumpe ausgebildete Pumpe 47. Der Pumpeneinlaß ist durch einen Pumpenschlauch 48 mit einem am Behälterboden 43 vorgesehenen Auslaß 49 oder Ablauf verbunden. Der Pumpenauslaß ist über einen weiteren Pumpenschlauch 50, der durch eine Öffnung der Wand 41 hindurchführt, mit dem Schlauchkonnektor 13 verbunden. Die elektrischen Anschlüsse der Batterie 46 und der Pumpe 47 sind über Kabel mit der Schaltungsplatine 35 verbunden. Diese Kabel führen durch dieselbe Öffnung hindurch, wie der Pumpenschlauch 50.

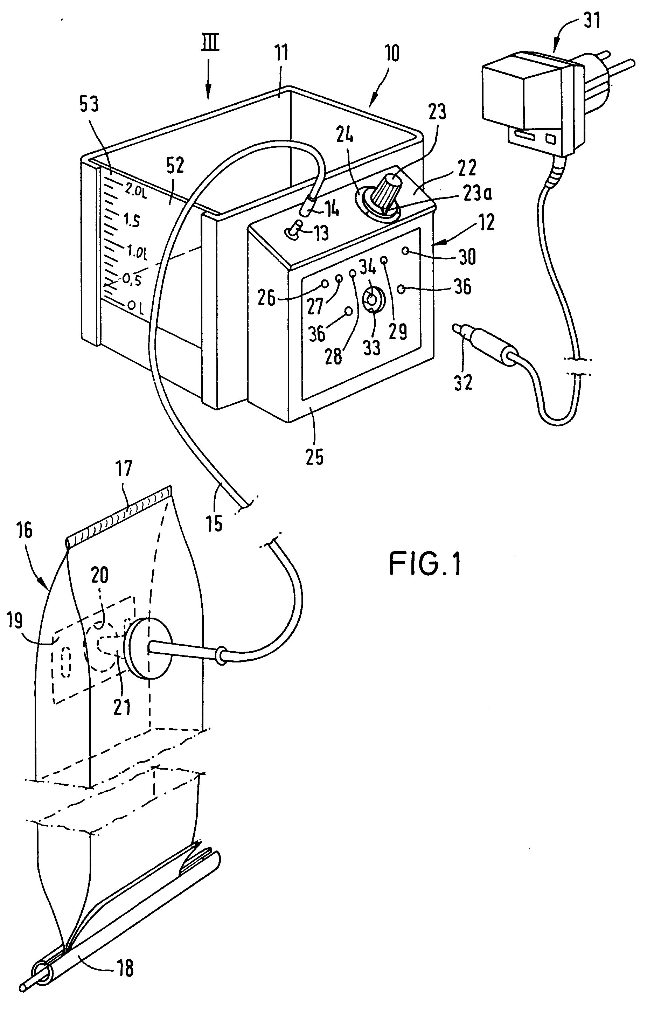

Gemäß Fig. 1 ist das Irrigationsgerät 10 an einer Seitenwand mit einem Sichtfenster 52 versehen, das eine Graduierung 53 zur Ermittlung des Füllstandes aufweist. Bei Benutzung des Gerätes wird vorgewärmte Spülflüssigkeit in den Flüssigkeitsbehälter 51 eingefüllt. Die Temperatur wird von dem Temperaturfühler 42 ermittelt und der Temperaturbereich wird an den Kontrolleuchten 26, 27, 28 angezeigt. Der Spülschlauch 15 wird mit dem Schlauchkonnektor 13 und mit dem Konus 21 des Beutels 16 verbunden, nachdem der Beutel am Körper des Patienten befestigt worden ist. Dann wird das Gerät 10 mit dem Drehknopf 23 eingeschaltet, wobei die Kontrolleuchte 30 zu leuchten beginnt. Durch Weiterdrehung des Drehknopfes 23 wird die Pumpe 47 in Betrieb gesetzt und es entsteht ein der Pumpendrehzahl entsprechender Spüldruck, mit dem der Darm gespült werden kann. Die Verschlußvorrichtung 18 kann von dem Beutel 16 abgenommen werden, um Spülflüssigkeit und Darminhalt sogleich in ein Toilettenbecken ablaufen zu lassen.

## Patentansprüche

1. Elektrische Irrigationsvorrichtung mit einem Flüssigkeitsbehälter (11), der an einer Wand (41) ein Schaltungsgehäuse (12) aufweist, und mit einer Kreiselpumpe (47) und einer Batterie (46), wobei die Saugseite der Kreiselpumpe mit einem Auslaß (49) des Flüssigkeitsbehälters (11) und die Druckseite der Kreiselpumpe mit einem Schlauchkonnektor (13) zum Anschließen eines externen Schlauchs (15) verbunden ist,
**dadurch gekennzeichnet,**
daß unter dem Flüssigkeitsbehälter (11) ein Aufnahmeraum (44) vorgesehen ist, der die Kreiselpumpe (47) und die Batterie (46) enthält, und daß der Auslaß (42) des Flüssigkeitsbehälters im Behälterboden (43) angeordnet ist.

2. Irrigationsvorrichtung nach Anspruch 1, dadurch gekennzeichnet, daß in der Nähe des Behälterbodens (43) ein Temperaturfühler (42) angeordnet ist, der mit einer im Schaltungsgehäuse (12) enthaltenen Auswerteschaltung verbunden ist, welche eine aus mindestens zwei Lampen (26-30) bestehende Anzeigevorrichtung mit Gut-Schlecht-Anzeige steuert.

3. Irrigationsvorrichtung nach Anspruch 1 oder 2, dadurch gekennzeichnet, daß das Schaltungsgehäuse (12) einen abnehmbaren Deckel (25) aufweist, an dem eine Schaltungsplatine (35) befestigt ist.

4. Irrigationsvorrichtung nach Anspruch 3, dadurch gekennzeichnet, daß an einer Wand des Schaltungsgehäuses ein Drehknopf (23) zum Einschalten der Pumpe angeordnet ist, der zugleich zum Einstellen der Pumpengeschwindigkeit dient.

5. Irrigationsvorrichtung nach Anspruch 3 oder 4, dadurch gekennzeichnet, daß eine Steckbuchse (34) für den Anschluß eines Netzgerätes (31) an die Batterie (46) an der Schaltungsplatine (35) angebracht und durch eine Öffnung (33) des Deckels (25) zugänglich ist.

6. Irrigationsvorrichtung nach einem der Ansprüche 3 bis 5, dadurch gekennzeichnet, daß Anzeige- und Kontrolleuchten ausschließlich an der Schaltungsplatine (35) angebracht sind und durch Löcher des Deckels (25) vorstehen.

7. Irrigationsvorrichtung nach einem der Ansprüche 1 bis 6, dadurch gekennzeichnet, daß der an dem Schaltungsgehäuse (12) befestigte Schlauchkonnektor (13) ein Lüer-Lock-Konnektor ist.

8. Irrigationsvorrichtung nach einem der Ansprüche 1 bis 7, dadurch gekennzeichnet, daß der Flüssigkeitsbehälter (11) ein mit einer Graduierung (53) versehenes Sichtfenster (52) aufweist.

9. Irrigationsvorrichtung nach Anspruch 4, dadurch gekennzeichnet, daß der Schlauchkonnektor (13) und der Drehknopf (23) an einer Dachwand (22) des Schaltungsgehäuses (12) angeordnet sind.

10. Irrigationsvorrichtung nach Anspruch 9, dadurch gekennzeichnet, daß die Dachwand (22) pultförmig schräg verläuft.
